# EUROPÄISCHE PATENTSCHRIFT

(11) **EP 0 681 216 B1**
(45) Veröffentlichungstag und Bekanntmachung des Hinweises auf die Patenterteilung: **15.12.1999**
(21) Anmeldenummer: 95106115.9
(22) Anmeldetag: 24.04.1995
(51) Int. Cl.: G03C 7/34, G03C 7/30, C07D 263/54

(54) **Fotografisches Farbentwicklungsverfahren**
Photographic colour developing process
Procédé photographique de développement en couleurs

(30) Priorität: 05.05.1994 DE 4415776
(43) Veröffentlichungstag der Anmeldung: 08.11.1995
(73) Patentinhaber: Agfa-Gevaert AG, 51373 Leverkusen (DE)
(72) Erfinder: Schenk, Günther, Dr., D-51467 Bergisch Gladbach (DE)

(56) Entgegenhaltungen:
- EP-A- 0 193 051

## Beschreibung

Die Erfindung betrifft ein fotografisches Farbentwicklungsverfahren, bei dem neue Cyankuppler verwendet werden.

Bei den erfindungsgemäß verwendeten Cyankupplern handelt es sich um Verbindungen der Formel I (4-Hydroxy-benzoxazole) 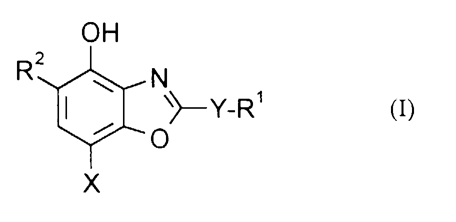 Verschiedene 4-Hydroxy-benzoxazole sind zwar als organische Verbindungen aus der Literatur bekannt, nicht jedoch als fotografische Kupplungskomponenten zur Erzeugung von blaugrünen Farbstoffchromophoren.

Es ist bekannt, farbige fotografische Bilder durch chromogene Entwicklung herzustellen, d.h. dadurch, daß man bildmäßig belichtete Silberhalogenidemulsionsschichten in Gegenwart geeigneter Farbkuppler mittels geeigneter farbbildender Entwicklersubstanzen-sogenannter Farbentwickler- entwickelt, wobei das in Übereinstimmung mit dem Silberbild entstehende Oxidationsprodukt der Entwicklersubstanzen mit dem Farbkuppler unter Bildung eines Farbstoffbildes reagiert. Als Farbentwickler werden gewöhnlich aromatische, primäre Aminogruppen enthaltende Verbindungen, insbesondere vom p-Phenylendiamintyp, verwendet.

An die Farbkuppler, sowie an die daraus durch chromogene Entwicklung erhaltenen Farbstoffe werden in der Praxis eine Reihe von Anforderungen gestellt. So soll die Kupplungsgeschwindigkeit der Farbkuppler mit dem Oxidationsprodukt des Farbentwicklers möglichst groß sein. Farbkuppler und die daraus erhaltenen Farbstoffe müssen hinreichend stabil sein gegenüber Licht, erhöhter Temperatur und Feuchtigkeit. Dies gilt sowohl für frisches Material, als auch für verarbeitetes Material. Beispielsweise darf der in den Bildweißen des verarbeiteten Materials noch vorhandene restliche Kuppler nicht vergilben. Außerdem sollen die Farbstoffe hinreichend beständig sein gegenüber gasförmigen reduzierenden oder oxidierenden Agentien. Sie müssen ferner diffusionsfest verankert sein und sollen sich bei der chromogenen Entwicklung als möglichst feines Korn abscheiden. Die mechanischen Eigenschaften der Schichten dürfen durch die Farbkuppler nicht beeinträchtigt werden. Schließlich müssen die aus den Farbkupplern bei der chromogenen Entwicklung entstehenden Farbstoffe eine günstige Absorptionskurve aufweisen mit einem Maximum, das der Farbe des jeweils gewünschten Teilbildes entspricht, und möglichst geringen Nebenabsorptionen. So soll ein Cyanfarbstoff im Idealfall rotes Licht absorbieren und grünes sowie blaues Licht weitgehend durchlassen. Außerdem sollen die Absorptionsmaxima der Farbstoffe sowohl bei Colorumkehr- wie auch bei Colornegativfilmen möglichst mit dem Sensibilisierungsmaxima der zum Kopieren verwendeten Colorpapiermaterialien übereinstimmen.

Als Cyankuppler, d.h. als Farbkuppler, die zur Erzeugung des blaugrünen Teilbildes geeignet sind, werden im allgemeinen Verbindungen verwendet, die sich von Phenol oder α-Naphthol ableiten.

Dokument EP-A-0 193 051 beschreibt ein fotographisches Farbentwicklungsverfahren, bei dem ein fotographisches Material verwendet wird, wobei einer Silberhalogenidemulsionsschicht ein 5-Hydroxy-6-acylamino-benzoxazol-2-on Kuppler zugeordnet ist.

Es wurde gefunden, daß die nachstehend beschriebenen Verbindungen der Formel I sich hervorragend als Cyankuppler eignen, die als Ergebnis der chromogenen Kupplung Farbstoffe mit z.T. brillanterer Nuance (verglichen mit bekannten Cyankupplern) liefern.

Gegenstand der Erfindung ist ein fotografisches Farbentwicklungsverfahren, bei dem ein farbfotografisches Aufzeichnungsmaterial mit mindestens einer bildmäßig belichteten Silberhalogenidemulsionsschicht in Gegenwart einer Farbkupplerverbindung und einer Farbentwicklerverbindung entwickelt wird, dadurch gekennzeichnet, daß die Farbkupplerverbindung der Formel I entspricht 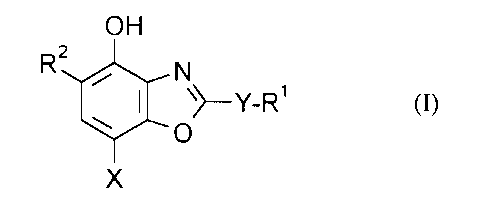 worin bedeuten
- R¹, R²: ein H-Atom oder einen Substituenten;
- X: ein H-Atom oder einen sonstigen bei der chromogenen Entwicklung abspaltbaren Rest;
- Y: eine chemische Bindung, einen Arylenrest, einen Hetarylenrest oder eine der folgenden Gruppen, die über eins ihrer N-Atome an den Benzoxazolring gebunden ist: -NH-CO-, -NH-SO₂-, -NH-CO-NH-, -NH-SO₂-NH-, -NH-CO-O-; wobei durch R¹ oder R² dargestellter Substituent einen Beitrag zur Ballastierung des Cyankupplers und/oder des daraus erzeugten Farbstoffes Leistet.

Gegenstand der Erfindung ist auch ein farbfotographishes Aufzeichnungsmaterial mit mindestens einer Lichtempfindlichen Silberhalogenidemulsionsschicht, der ein Farbkuppler der Formel I zugeordnet ist.

Es gibt keine spezielle Einschränkung für die Substituenten R¹ und R², typische Beispiele sind z.B. Alkyl, Aryl, Anilino, Acylamino, Sulfonamido, Alkylthio, Arylthio, Alkenyl, und außer Halogen, auch Substituenten wie Cycloalkenyl, Alkinyl, Heterocyclyl, Sulfonyl, Sulfinyl, Phosphonyl, Acyl, Carbamoyl, Sulfamoyl, Cyano, Alkoxy, Sulfonyloxy, Aryloxy, Heterocyclyloxy, Siloxy, Acyloxy, Carbamoyloxy, Amino, Alkylamino, Imido, Ureido, Sulfamoylamino, Alkoxycarbonylamino, Aryloxycarbonylamino, Alkoxycarbonyl, Aryloxycarbonyl, Heterocyclylthio, Thioureido, Carboxy, Nitro, Sulfonat, Spirosubstituenten, sowie Brückenkohlenwasserstoffreste, mit der Maßgabe, daß R¹ nicht für Halogen steht.

Alkyl-Sustituenten für R¹ bzw. R² sind hier vorzugsweise Reste mit 1 bis 32 C-Atomen, geradkettig oder verzweigt, unsubstituiert oder substituiert, z.B. substituiert mit Alkoxy, Aryloxy oder Acyl.

Aryl-Substituenten für R¹ bzw. R² sind hier vorzugsweise Phenyl bzw. substituierte Phenylreste.

Acylamino-Substituenten für R¹ bzw. R² sind hier bevorzugt Alkylcarbonylamino, Arylcarbonylamino, Hetarylcarbonylamino, Ureido.

Sulfonamido-Substituenten für R¹ bzw. R² sind hier bevorzugt Alkylsulfonylamino, Arylsulfonylamino, Hetarylsulfonylamino.

Bei den Alkylthio- und Arylthio-Substituenten für R¹ bzw. R² sind die Alkyl- bzw. Aryl-Fragmente in ihrer Bandbreite identisch mit der Beschreibung der Substituenten Alkyl und Aryl (für R¹ und R²).

Alkenyl-Substituenten für R¹ bzw. R² enthalten 2 bis 32 Kohlenstoffatome; der Alkenyl-Substituent kann geradkettig oder verzweigt sein.

Cycloalkyl- und Cycloalkenyl-Substituenten für R¹ bzw. R² enthalten 3 bis 12 C-Atome, vorzugsweise 5 bis 7 C-Atome.

Sulfonyl-Substituenten für R¹ bzw. R² sind z.B. jede Art Alkylsulfonyl, Arylsulfonyl oder Hetarylsulfonyl.

Sulfinyl-Substituenten für R¹ bzw. R² sind z.B. jede Art Alkylsulfinyl, Arylsulfinyl oder Hetarylsulfinyl.

Phosphonyl-Substituenten für R¹ bzw. R² sind z.B. jede Art Alkylphosphonyl, Alkoxyphosphonyl, Aryloxyphosphonyl oder Arylphosphonyl.

Acyl-Substituenten für R¹ bzw. R² sind z.B. jede Art Alkylcarbonyl oder Arylcarbonyl.

Carbamoyl-Substituenten für R¹ bzw R² sind z.B. jede Art Alkylcarbamoyl, Arylcarbamoyl oder Hetarylcarbamoyl.

Sulfamoyl-Substituenten für R¹ bzw. R² sind z.B. jede Art Alkylsulfamoyl, Arylsulfamoyl oder Hetarylsulfamoyl.

Acyloxy-Substituenten für R¹ bzw. R² sind z.B. jede Art Alkylcarbonyloxy oder Arylcarbonyloxy.

Carbamoyloxy-Substituenten für R¹ bzw. R² sind z.B. jede Art Alkylcarbamoyloxy oder Arylcarbamoyloxy.

Ureido-Substituenten für R¹ bzw. R² sind z.B. jede Art Alkylureido, Arylureido oder Hetarylureido.

Sulfamoylamino-Substituenten für R¹ bzw. R² sind z.B. jede Art von Alkylsulfamoylamino oder Arylsulfamoylamino.

Heterocyclische Substituenten für R¹ bzw. R² sind vorzugsweise 5- bis 7-Ringheterocyclen; typische Beispiele sind: 2-Furyl, 2-Thienyl, 2-Pyrimidinyl, 2-Benzthiazolyl, 1-Pyrrolyl, 1-Tetrazolyl.

Heterocyclyloxy-Substituenten für R¹ bzw. R² sind vorzugsweise über ein O-Atom gebundene 5- bis 7-Ringheterocyclen; typische Beispiele sind: 3,4,5,6-Tetrahydropyranyl-2-oxy, 1-Phenyltetrazol-5-oxy.

Heterocyclylthio-Substituenten für R¹ bzw. R² sind vorzugsweise über ein S-Atom gebundene 5- bis 7-Ringheterocyclen; typische Beispiele sind: 2-Pyridylthio, 2-Benzthiazolylthio, 2,4-Diphenoxy-1,3,5-triazol-6-thio.

Imido-Substituenten für R¹ bzw. R² sind z.B. Succinimido, 3-Heptadecylsuccinimido, Phthalimido, Glutarimido.

Spiro-Substituenten für R¹ bzw. R² sind z.B. Spiro[3,3]heptan-1-yl.

Verbrückte Kohlenwasserstoff-Substituenten für R¹ bzw. R² sind z.B Bicyclo[2,2,1]heptan-1-yl, Tricyclo[3,2,1,1]decan-1-yl, 7,7-Dimethyl-bicyclo[2,2,1]heptan-1-yl.

Ein durch X dargestellter bei der chromogenen Entwicklung abspaltbarer Rest X kann beispielsweise sein: H, ein Halogenatom, wie Cl, Br oder F, Substituenten wie: Alkoxy, Aryloxy, Heterocyclyloxy, Acyloxy, Sulfonyloxy, Alkoxycarbonyloxy, Aryloxycarbonyloxy, Alkyloxalyloxy, Alkoxyoxalyloxy, Alkylthio, Arylthio, Heterocyclylthio, Alkyloxythiocarbonylthio, Acylamino, Sulfonamido, ein N-haltiger Heterocyclus, der über ein N-Atom mit dem Kupplerrest verknüpft ist, Alkoxycarbonylamino, Aryloxycarbonylamino, Carboxyl, -N=N-Aryl.

Je nach dem Substituenten X an der zur Kupplung befähigten Position kuppeln die erfindungswesentlichen Cyankuppler entweder nach dem 4-Äquivalentprinzip oder als 2-Äquivalentkuppler, wobei der abgespaltene nukleophile Rest spezielle fotografische Wirkungen hervorrufen kann. Es kann zudem ein nukleophiler Rest abgespalten werden, der seine fotografische Wirkung erst nach Abspaltung eines Zwischengliedes entfaltet.

Eine in Formel I durch X dargestellte unter den Bedingungen der chromogenen Entwicklung abspaltbare Gruppe ist beispielsweise entweder selbst der Rest einer fotografisch wirksamen Verbindung oder eine Gruppe, die nach Abspaltung aus der Kupplungsstelle des Kupplers bei dessen Kupplung mit dem Oxidationsprodukt des Silberhalogenidentwicklungsmittels befähigt ist, erst in einer Folgereaktion einen daran gebundenen Rest einer fotografisch wirksamen Verbindung freizusetzen. Eine solche Gruppe wird auch als Zeitsteuerglied bezeichnet, weil der daran gebundene Rest der fotografisch wirksamen Verbindung in vielen Fällen verzögert freigesetzt wird und erst dann wirksam werden kann. Bekannte

Zeitsteuerglieder sind beispielsweise eine Gruppe 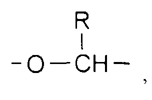 wobei das O-Atom an die Kupplungsstelle des Kupplers und das C-Atom an ein N-Atom einer fotografisch wirksamen Verbindung gebunden ist (z.B. DE-A-28 03 145), eine Gruppe, die nach Abspaltung vom Kuppler einer intramolekularen nukleophilen Verdrängungsreaktion unterliegt und hierbei die fotografisch wirksame Verbindung freisetzt (z.B. DE-A-28 55 697), eine Gruppe, in der nach Abspaltung vom Kuppler eine Elektronenübertragung entlang eines konjugierten Systems stattfinden kann, wodurch die fotografische wirksame Verbindung freigesetzt wird (z.B. DE-A-31 05 026), oder eine Gruppe 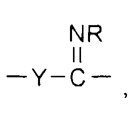 worin Y (z.B. -O-) an die Kupplungsstelle des Kupplers und das C-Atom an ein Atom der fotografisch wirksamen Verbindung gebunden ist und worin R beispielsweise für Aryl steht (z.B. EP-A-0 127 063). Das Zeitsteuerglied kann auch eine Gruppe sein, die nach Abspaltung aus der Kupplungsstelle des Kupplers selbst eine Kupplungsreaktion oder eine Redoxreaktion eingehen kann und als Folge einer solchen Reaktion die an sie gebundene fotografisch wirksame Verbindung freisetzt.

Bei der unter den Bedingungen der chromogenen Entwicklung abspaltbaren Gruppe handelt es sich beispielsweise um eine organische Gruppe, die in der Regel über ein Sauerstoff-, Schwefel- oder Stickstoffatom an die Kupplungsstelle des Kupplermoleküls angeknüpft ist. Falls es sich bei der abspaltbaren Gruppe um eine cyclische Gruppe handelt, kann die Anknüpfung an die Kupplungsstelle des Kupplermoleküls entweder direkt über ein Atom, das Bestandteil eines Ringes ist, z.B. ein Stickstoffatom, oder indirekt über ein zwischengeschaltetes Bindeglied erfolgt sein. Derartige abspaltbare Gruppen sind in großer Zahl bekannt, z.B. als Fluchtgruppen von 2-Äquivalentkupplern.

Beispiele von über Sauerstoff angeknüpften abspaltbaren Gruppen entsprechen der Formel

-O-R³ ,

worin R³ für einen acyclischen oder cyclischen organischen Rest steht, z.B. für Alkyl, Aryl, eine heterocyclische Gruppe oder Acyl, das sich beispielsweise ableitet von einer organischen Carbon- oder Sulfonsäure.

Bei besonders bevorzugten abspaltbaren Gruppen dieser Art bedeutet R³ eine gegebenenfalls substituierte Phenylgruppe. Solche Gruppen sind beispielsweise beschrieben in US-A-3 408 194, DE-A-24 56 076.

Beispiele von über Stickstoff angeknüpften abspaltbaren Gruppen sind in den folgenden deutschen Offenlegungsschriften (DE-A-) beschrieben:
20 57 941, 21 63 812, 22 13 461, 22 19 917, 22 61 361, 22 63 875, 23 18 807, 23 29 587, 23 44 155, 23 63 675, 24 33 812, 24 41 779, 24 42 703, 25 28 638, 25 28 860, 26 37 817, 28 18 373, 30 20 416.

Hierbei handelt es sich durchweg um 5- oder 6-gliedrige heterocyclische Ringe, die über ein Ringstickstoffatom mit der Kupplungsstelle des Kupplers verbunden sind. Die heterocyclischen Ringe enthalten vielfach benachbart zudem die Bindung an das Kupplermolekül vermittelnden Stickstoffatom aktivierende Gruppen, z.B. Carbonyl- oder Sulfonylgruppen oder Doppelbindungen.

Bei der fotografisch wirksamen Verbindung kann es sich um einen Entwicklungsinhibitor, einen Bleichinhibitor, einen Entwicklungsbeschleuniger, ein silberkeimbildendes "Nukleierungsmittel", eine lösliche ausentwicklungsfördernde Mercaptanverbindung, einen Stabilisator, einen Weißkuppler, einen Scavanger, ein Elektronentransferagens, z.B. vom Phenidontyp oder eine Farbkupplerverbindung handeln.

Als Entwicklungsinhibitoren sind insbesondere solche aus der Reihe der Benzotriazole, der Thienotriazole, der nichtkondensierten monocyclischen 1,2,3-Triazole, der 3-Alkylthio-1,2,4-triazole, der 5-Mercapto-1-alkyl- oder 5-Mercapto-1-amyltetrazole sowie der 2-Mercapto-5-alkylthio-1,3,4-thiadiazole zu nennen.

Die erfindungswesentlichen Farbkuppler können weiterhin mit einem Ballastrest ausgestattet sein, beispielsweise über R¹ und/oder R². Als Ballastreste sind solche Reste anzusehen, die es ermöglichen oder erleichtern, die erfindungswesentlichen Farbkuppler in den üblicherweise bei fotografischen Materialien verwendeten hydrophilen Kolloiden diffusionsfest einzulagern. Hierzu sind vorzugsweise organische Reste geeignet, die im allgemeinen geradkettige oder verzweigte aliphatische Gruppen und gegebenenfalls auch carbocyclische oder heterocyclische aromatische Gruppen mit im allgemeinen 8 bis 20 C-Atome enthalten.

Beispiele für erfindungswesentliche Cyankuppler sind im folgenden aufgeführt.

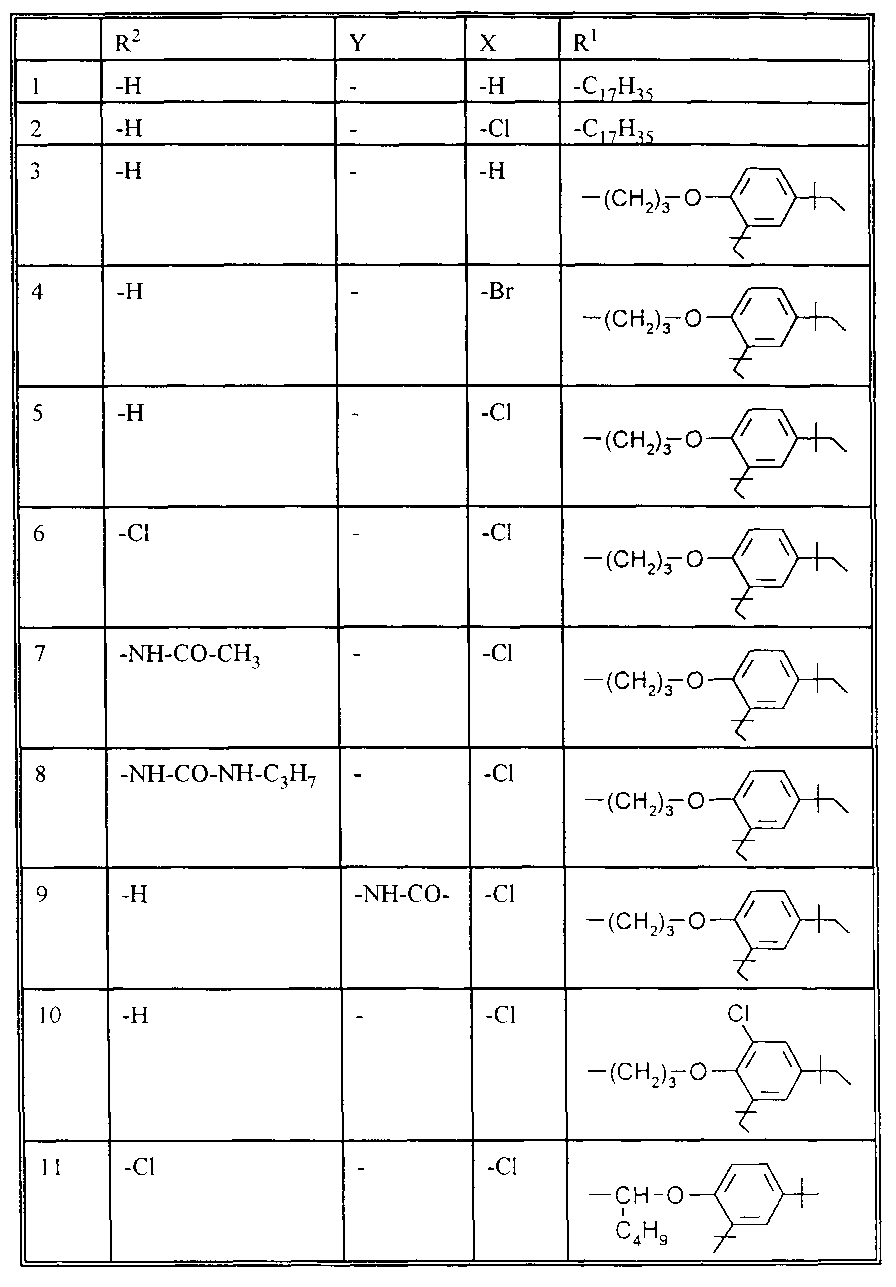

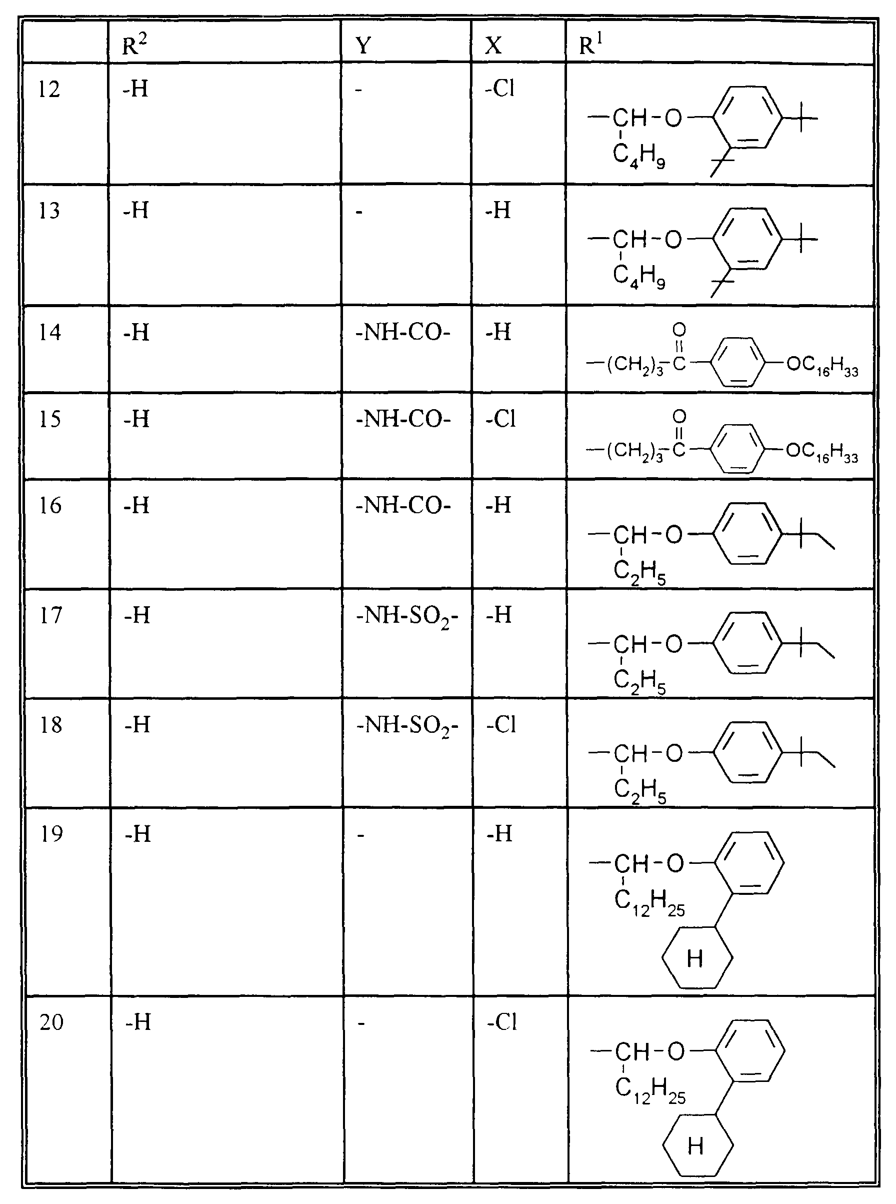

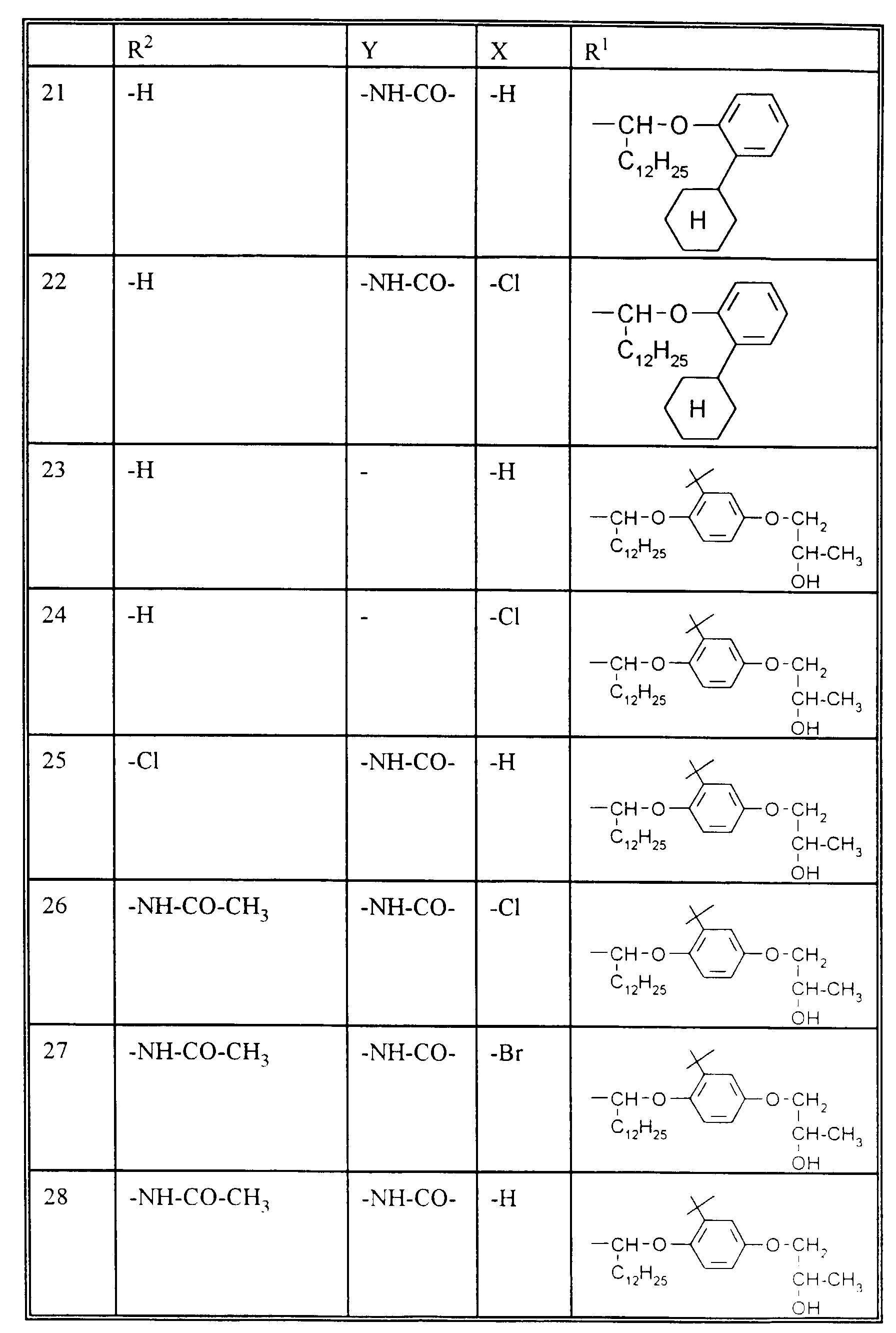

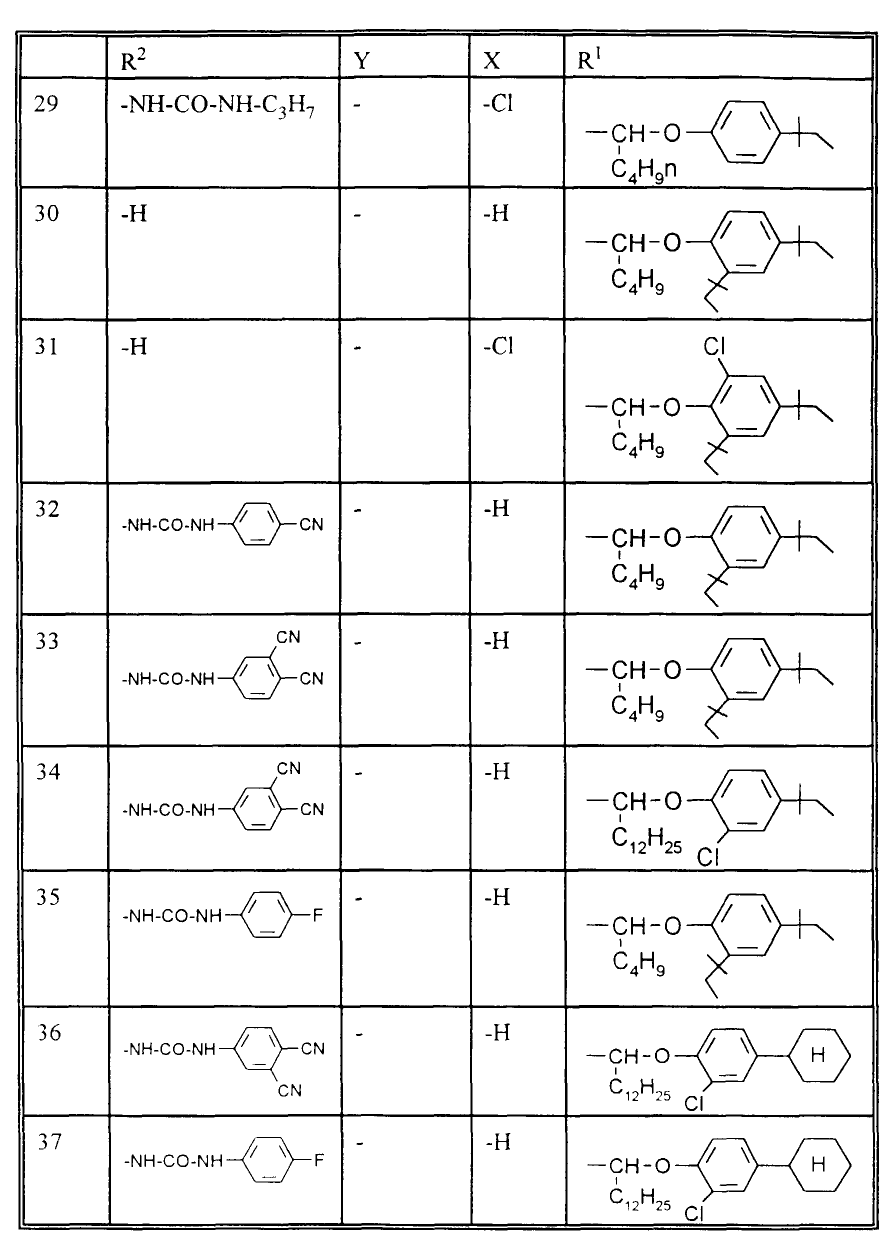

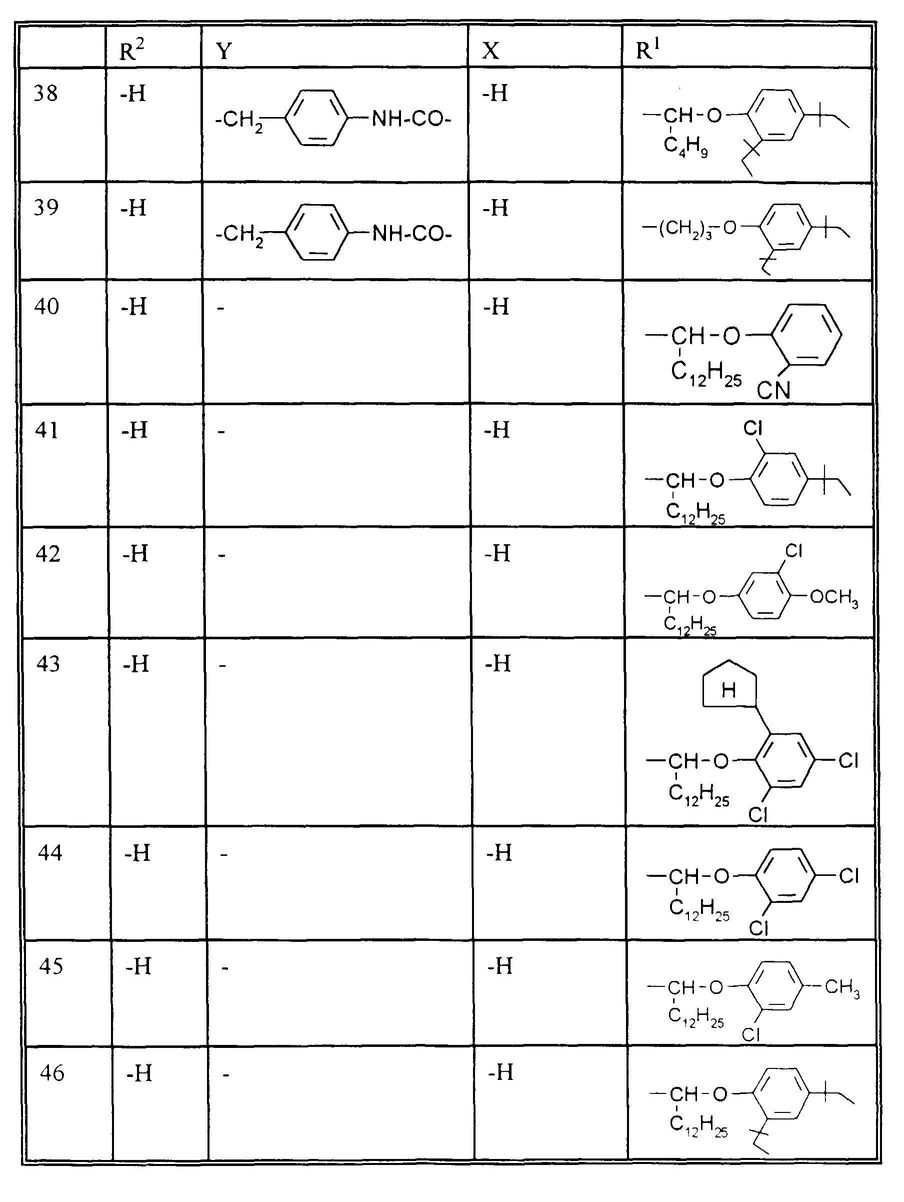

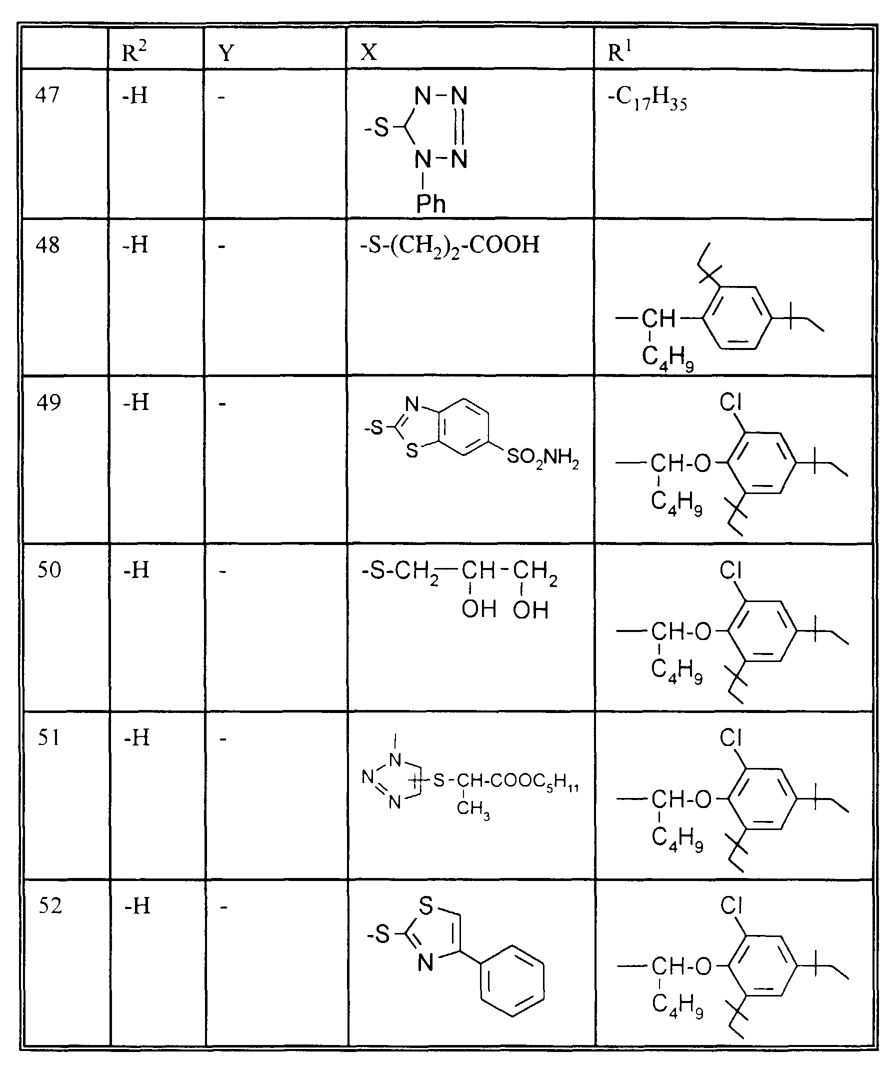

Zur Synthese der erfindungswesentlichen Farbkuppler stehen mehrere Methoden zur Verfügung (siehe allgemeines Schema 1) 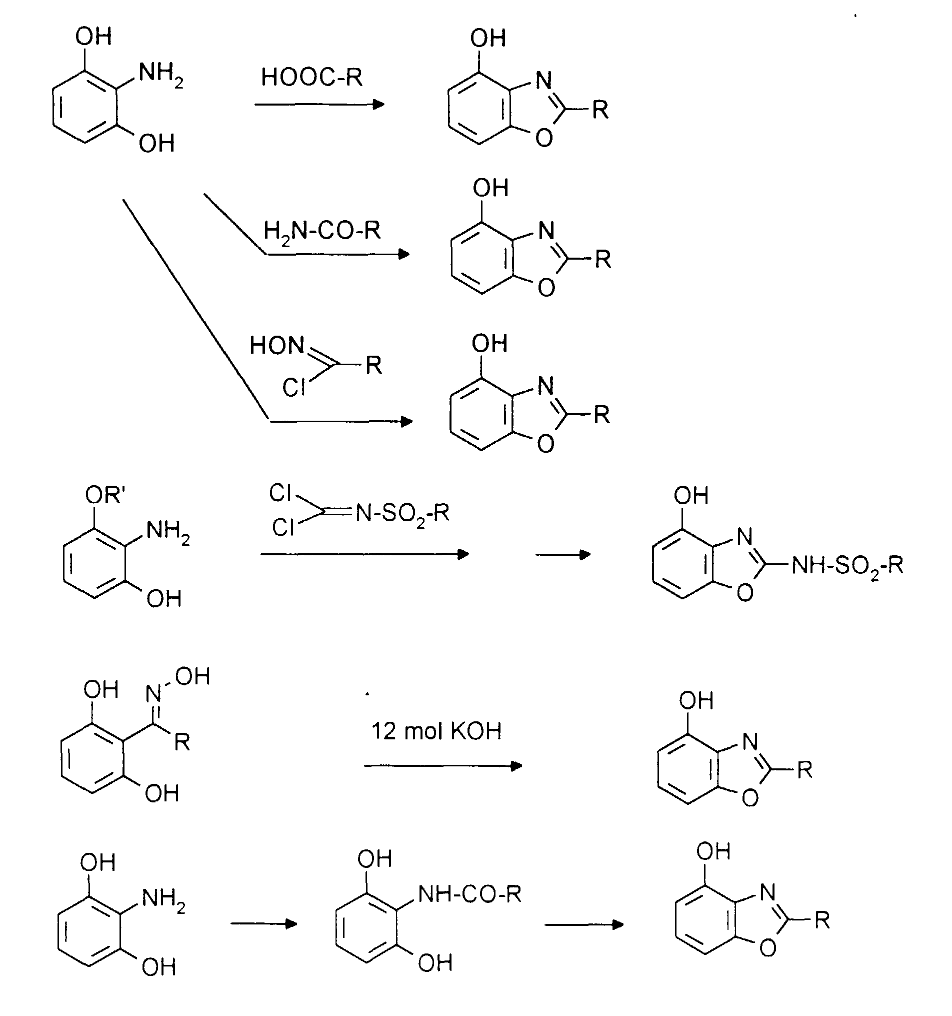
1. Kondensation von 2-Aminophenolen mit Carbonsäuren in PPE/CHCl₃ oder ohne Solvens;
2. Kondensation von 2-Aminophenolen mit Carbonamiden;
3. 2-stufig: Umsetzung von 2-Aminophenolen zu 2-Carbamoylphenol mit nachfolgender sauer katalysierter Kondensation zum Benzoxazol;
4. Cyclisierung von 2-Aminophenolen mit Hydroximsäurechloriden.
5. Ausgehend von 2',6'-Dihydroxy-aceto- bzw. -benzophenonen zu den entsprechenden Oximen, dann Cyclisierung mit 12 mol KOH in MeOH/H₂O (Variation R1 sehr eng; stets mitgebildetes 4-Hydroxy-3-alkyl(aryl)-benzisoxazol stört).
6. Umsetzung von 2-Aminophenol mit N-Dichlormethylensulfonamid zu 2-Sulfonylbenzoxazolen

### Herstellungsbeispiele

### Cyankuppler 1

24,00 g 2,6-Dihydroxyanilin-hydrochlorid werden zusammen mit 43,41 g Stearinsäureamid verschmolzen und 20 min bei 200°C gerührt. Nach Abkühlung auf 80°C wird mit 240 ml Ethanol verrührt, auf Raumtemperatur abgekühlt, abgesaugt und getrocknet. Umkristallisation aus Methanol mit Aktivkohle liefert 32,7 g Cyankuppler 1.

### Cyankuppler 2

18,7 g Cyankuppler 1 werden in 100 ml trockenem Methylenchlorid verrührt, dann bei Raumtemperatur 6,8 g frisch destilliertes Sulfurylchlorid, gelöst in 20 ml Methylenchlorid, zugetropft. Dann wird 60 min bei Raumtemperatur nachgerührt, dann auf 2/3 des Volumens eingeengt, mit 300 ml Methanol versetzt, kurz am Rückfluß gehalten, abgekühlt und abgesaugt. Man erhält 16 g Rohprodukt. Nach 2-maligem Umkristallisieren aus Methanol werden 14,2 g Cyankuppler 2 erhalten.

### Cyankuppler 3

### 3.1 2-Carbamoyl-Resorcin A:

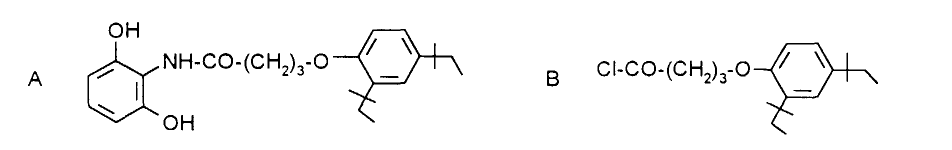

64,6 g 2-Aminoresorcin-hydrochlorid werden in 400 ml Acetonitril verrührt, 160,8 g Triethylamin zugegeben und auf 70°C gebracht. Dann werden langsam 135,3 g Säurechlorid B, gelöst in 120 ml Acetonitril, zugetropft und ca. 3 h bei 70°C gerührt.

Nach Abkühlung wird in 2 l salzsaures Eiswasser eingerührt und abgesaugt, mit Wasser nachgewaschen und getrocknet. Man erhält 90 g Rohprodukt. Nach Umkristallisation aus Ethanol mit Aktivkohle werden 72,4 g reines A erhalten.

### 3.2 Cyankuppler 3

42,73 g Verbindung A werden in 1000 ml Toluol gelöst und 40 g p-Toluolsulfonsäure zugesetzt und ca. 5 h am Wasserabscheider gekocht. Darauf wird das Lösungsmittel abdestilliert und das verbleibende Öl in 500 ml Essigester aufgenommen, 4-mal mit 250 ml Wasser gewaschen; die organische Phase getrocknet und eingeengt. Der Rückstand wird in 80 ml CH₃CN/Aktivkohle 2-mal umkristallisiert. Ausbeute: 27 g Cyankuppler 3.

Bei dem erfindungsgemäßen Verfahren wird ein farbfotografisches Material, das wenigstens eine bildmäßig belichtete Silberhalogenidemulsion enthält, mit einer Farbentwicklerverbindung vom p-Phenylendiamintyp entwickelt. Die erfindungswesentlichen Cyankuppler können dabei in räumlicher und spektraler Zuordnung zu einer lichtempfindlichen Silberhalogenidemulsionsschicht in dem Material enthalten sein.

Unter räumlicher Zuordnung ist dabei zu verstehen, daß der Farbkuppler sich in einer solchen räumlichen Beziehung zu der betreffenden Silberhalogenidemulsionsschicht befindet, daß eine Wechselwirkung zwischen ihnen möglich ist, die eine bildmäßige Übereinstimmung zwischen dem bei der Entwicklung gebildeten Silberbild und dem aus dem Farbkuppler erzeugten Farbbild zuläßt. Dies wird in der Regel dadurch erreicht, daß der Farbkuppler in der Silberhalogenidemulsionsschicht selbst enthalten ist oder in einer hierzu benachbarten gegebenenfalls nichtlichtempfindlichen Bindemittelschicht.

Unter spektraler Zuordnung ist zu verstehen, daß die Spektralempfindlichkeit der betreffenden lichtempfindlichen Silberhalogenidemulsionsschicht und die Farbe des aus dem räumlich zugeordneten Farbkuppler erzeugten Teilfarbenbildes in einer bestimmten Beziehung zueinander stehen, wobei normalerweise jeder der Spektralempfindlichkeiten (Rot, Grün, Blau) eine andere Farbe des betreffenden Teilfarbenbildes (z.B. Blaugrün, Purpur, Gelb) zugeordnet ist Entsprechend der aus den erfindungswesentlichen Cyankupplern gebildeten Farbe werden diese vorzugsweise einer rotsensibilisierten Silberhalogenidemulsionsschicht zugeordnet.

Das Material kann weiterhin von Kupplern verschiedene Verbindungen enthalten, die beispielsweise einen Entwicklungsinhibitor, einen Entwicklungsbeschleuniger, einen Bleichbeschleuniger, einen Entwickler, ein Silberhalogenidlösungsmittel, ein Schleiermittel oder ein Antischleiermittel in Freiheit setzen können, beispielsweise sogenannte DIR-Hydrochinone und andere Verbindungen, wie sie beispielsweise in US-A-4 636 546, 4 345 024, 4 684 604 und in DE-A-31 45 640, 25 15 213, 24 47 079 und in EP-A-198 438 beschrieben sind. Diese Verbindungen erfüllen die gleiche Funktion wie die DIR-, DAR- oder FAR-Kuppler, außer daß sie keine Kupplungsprodukte bilden.

Hochmolekulare Farbkuppler sind beispielsweise in DE-C-1 297 417, DE-A-24 07 569, DE-A-31 48 125, DE-A-32 17 200, DE-A-33 20 079, DE-A-33 24 932, DE-A-33 31 743, DE-A-33 40 376, EP-A-27 284, US-A-4 080 211 beschrieben. Die hochmolekularen Farbkuppler werden in der Regel durch Polymerisation von ethylenisch ungesättigten monomeren Farbkupplern hergestellt. Sie können aber auch durch Polyaddition oder Polykondensation erhalten werden.

Die Einarbeitung der erfindungswesentlichen Farbkuppler in Silberhalogenidemulsionsschichten kann in der Weise erfolgen, daß zunächst von der betreffenden Verbindung eine Lösung, eine Dispersion oder eine Emulsion hergestellt und dann der Gießlösung für die betreffende Schicht zugefügt wird. Die Auswahl des geeigneten Lösungs- oder Dispersionsmittels hängt von der jeweiligen Löslichkeit der Verbindung ab.

Methoden zum Einbringen von in Wasser im wesentlichen unlöslichen Verbindungen durch Mahlverfahren sind beispielsweise in DE-A-26 09 741 und DE-A-26 09 742 beschrieben.

Hydrophobe Verbindungen können auch unter Verwendung von hochsiedenden Lösungsmitteln, sogenannten Ölbildnern, in die Gießlösung eingebracht werden. Entsprechende Methoden sind beispielsweise in US-A-2 322 027, US-A-2 801 170 und EP-A-0 043 037 beschrieben.

Anstelle der hochsiedenden Lösungsmittel können Oligomere oder Polymere, sogenannte polymere Ölbildner Verwendung finden.

Die Verbindungen können auch in Form beladener Latices in die Gießlösung eingebracht werden. Verwiesen wird beispielsweise auf DE-A-25 41 230, DE-A-25 41 274, DE-A-28 35 856, EP-A-0 014 921, EP-A-0 069 671, EP-A-0 130 115, US-A-4 291 113.

Die diffusionsfeste Einlagerung anionischer wasserlöslicher Verbindungen (z.B. von Farbstoffen) kann auch mit Hilfe von kationischen Polymeren, sogenannten Beizenpolymeren erfolgen.

Geeignete Ölbildner sind z.B. Phthalsäurealkylester, Phosphonsäureester, Phosphorsäureester, Citronensäureester, Benzoesäureester, Amide, Fettsäureester, Trimesinsäureester, Alkohole, Phenole, Anilinderivate und Kohlenwasserstoffe.

Beispiele für geeignete Ölbildner sind Dibutylphthalat, Dicyclohexylphthalat, Di-2-ethylhexylphthalat, Decylphthalat, Triphenylphosphat, Tricresylphosphat, 2-Ethylhexyldiphenylphosphat, Tricyclohexylphosphat, Tri-2-ethylhexylphosphat, Tridecylphosphat, Tributoxyethylphosphat, Trichlorpropylphosphat, Di-2-ethylhexylphenylphosphat, 2-Ethylhexylbenzoat, Dodecylbenzoat, 2-Ethylhexyl-p-hydroxybenzoat, Diethyldodecanamid, N-Tetradecylpyrrolidon, Isostearylalkohol, 2,4-Di-t-amylalkohol, Dioctylacetat, Glycerintributyrat, Glycerintributyrat, Isostearyllactat, Trioctylcitrat, N,N-Dibutyl-2-butoxy-5-t-octylanilin, Paraffin, Dodecylbenzol und Diisopropylnaphthalin.

Farbfotografische Negativmaterialien werden üblicherweise durch Entwickeln, Bleichen, Fixieren und Wässern oder durch Entwickeln, Bleichen, Fixieren und Stabilisieren ohne nachfolgende Wässerung verarbeitet, wobei Bleichen und Fixieren zu einem Arbeitsschritt zusammengefaßt sein können. Als Farbentwicklerverbindung lassen sich sämtliche Entwicklerverbindungen verwenden, die die Fähigkeit besitzen, in Form ihres Oxidationsproduktes mit den erfindungswesentlichen Farbkupplern zu Azomethinfarbstoffen zu reagieren. Geeignete Farbentwicklerverbindungen sind aromatische, mindestens eine primäre Aminogruppe enthaltende Verbindungen vom p-Phenylendiamintyp, beispielsweise N,N-Dialkyl-p-phenylendiamine wie N,N-Diethyl-p-phenylendiamin, 1-(N-Ethyl-N-methyl sulfonamidoethyl)-3-methyl-p-phenylendiamina, 1-(N-Ethyl-N-hydroxyethyl)-3-methyl-p-phenylendiamin und 1-( N-Ethyl-N-methoxyethyl)-3-methyl-p-phenylendiamin.

Weitere brauchbare Farbentwickler sind beispielsweise in J. Amer. Chem. Soc. 73, 3106 (1951) und G. Haist, Modern Photographic Processing, 1979, John Wiley and Sons, New York, Seite 545 ff. beschrieben.

Sofern es sich bei den erfindungswesentlichen Farbkupplern um alkalilösliche Verbindungen handelt, können sie auch (anstatt dem fotografischen Material) dem Farbentwickler zugesetzt werden.

Nach der Farbentwicklung kann ein saures Stoppbad oder eine Wässerung erfolgen.

Üblicherweise wird das Material unmittelbar nach der Farbentwicklung gebleicht und fixiert. Als Bleichmittel können z.B. Fe(III)-Salze und Fe(III)-Komplexsalze wie Ferricyanide, Dichromate, wasserlösliche Kobaltkomplexe verwendet werden. Besonders bevorzugt sind Eisen(III)-Komplexe von Aminopolycarbonsäuren, insbesondere z.B. von Ethylendiamintetraessigsäure, Propylendiamintetraessigsäure, Diethylentriaminpentaessigsäure, Nitrilotriessigsäure, Imidoessigsäure, N-Hydroxyethyl-ethylendiamintriessigsäure, Alkyliminodicarbonsäure und von entsprechenden Phosphonsäuren. Geeignet als Bleichmittel sind weiterhin Persulfate und Peroxide, z.B. Wasserstoffperoxid.

Auf das Bleichfixierbad oder Fixierbad folgt meist eine Wässerung, die vorteilhaft als Gegenstromwässerung ausgeführt sein kann oder aus mehreren Tanks mit eigener Wasserzufuhr bestehen kann.

Günstige Ergebnisse können bei Verwendung eines darauf folgenden Schlußbades, das keinen oder nur wenig Formaldehyd, erhalten werden.

Die Wässerung kann aber durch ein Stabilisierbad vollständig ersetzt werden, das üblicherweise im Gegenstrom geführt wird. Dieses Stabilisierbad übernimmt bei Formaldehydzusatz auch die Funktion eines Schlußbades.

Bei Farbumkehrmaterialien erfolgt zunächst eine Entwicklung mit einem Schwarz-Weiß-Entwickler, dessen Oxidationsprodudkt nicht zur Reaktion mit dem Farbkuppler befähigt ist. Es schließt sich eine diffuse Zweitbelichtung und dann Entwicklung mit einem Farbentwickler, Bleichen und Fixieren an.

### Beispiel 1

Ein farbfotografisches Aufzeichnungsmaterial wurde hergestellt (Schichtaufbau 1A - Vergleich), indem auf einen transparenten Schichtträger aus Cellulosetriacetat die folgenden Schichten in der angegebenen Reihenfolge aufgetragen wurden. Die Mengenangaben beziehen sich jeweils auf m². Für den Silberhalogenidauftrag werden die entsprechenden Mengen AgNO₃ angegeben. Die Silberhalogenidemulsion war auf 100 g AgNO₃ mit 0,1 g 4-Hydroxy-6-methyl-1,3,3a,7-tetraazainden stabilisiert.

### Schichtaufbau 1A (Vergleich)

- Schicht 1: (Antihaloschicht)
schwarzes kolloidales Silbersol mit
0,2 g Ag
1,2 g Gelatine
- Schicht 2: (Mikrat-Zwischenschicht)
Mikrat-Silberbromidiodidemulsion
(0,5 mol-% Iodid;
mittlerer Korndurchmesser 0,07 µm)
aus 0,25 g AgNO₃, mit
1,0 g Gelatine
- Schicht 3: (rotempfindliche Schicht
rotsensibilisierte Silberbromidiodidemulsion
(4,0 mol-% Iodid;
mittlerer Korndurchmesser 0,45 µm)
aus 5,35 g AgNO₃, mit
3,75 g Gelatine
1,33 g Cyankuppler XC-1
1,33 g Trikresylphosphat (TKP)
- Schicht 4: (Schutz- und Härtungsschicht
aus 0,68 g Gelatine
0,73 g Härtungsmittel
(CAS Reg. No. 65411-60-1)

Der verwendete Cyankuppler XC-1 hat die Formel

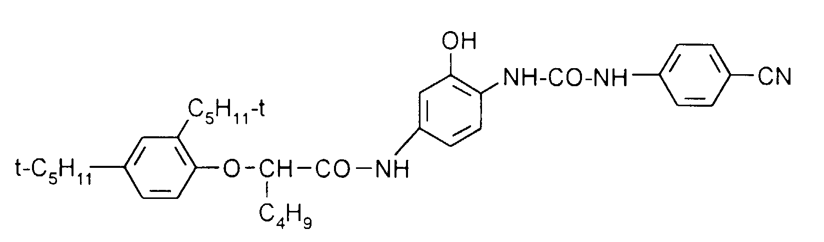

### Schichtaufbau 1B (gemäß Erfindung):

Wie Schichtaufbau 1A, jedoch mit folgender Änderung:
- Schicht 3: enthält anstelle Cyankuppler XC-1 1,32 g Cyankuppler 3

Die Entwicklung wurde nach Aufbelichtung eines Graukeils, wie in Brit. J. Phot, S. 597-598 beschrieben, durchgeführt.

Man erhält sowohl für Schichtaufbau 1A (Vergleich) wie für Schichtaufbau 1B (Erfindung) je einen blaugrünen Keil. Die Maximaldichten betragen 1,7 bzw. 1,92 gemessen hinter Rotfilter.

Bei Verwendung des erfindungswesentlichen Cyankuppler 3 zeichnet sich der Farbkeil durch hohe Farbreinheit bzw. Brillanz aus. Brillanz aus.

## Patentansprüche

1. Fotografisches Farbentwicklungsverfahren, bei dem ein farbfotografisches Aufzeichnungsmaterial mit mindestens einer bildmäßig belichteten Silberhalogenidemulsionsschicht in Gegenwart einer Farbkupplerverbindung und einer Farbentwicklerverbindung entwickelt wird, dadurch gekennzeichnet, daß die Farbkupplerverbindung der Formel I entspricht 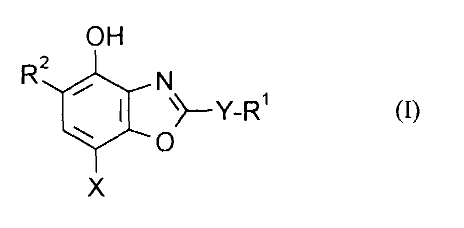 worin bedeuten
R¹, R² ein H-Atom oder einen Substituenten;
X ein H-Atom oder einen sonstigen bei der chromogenen Entwicklung abspaltbaren Rest;
Y eine chemische Bindung, einen Arylenrest, einen Hetarylenrest oder eine der folgenden Gruppen, die über eins ihrer N-Atome an den Benzoxazolring gebunden ist: -NH-CO-, -NH-SO₂-, -NH-CO-NH-, -NH-SO₂-NH-, -NH-CO-O-; wobei ein durch R¹ oder R² dargestellter Substituent einen Beitrag zur Ballastierung der Cyankupplers und/oder des daraus erzeugten Farbstoffes Leistet.

2. Farbfotografisches Aufzeichnungsmaterial mit mindestens einer lichtempfindlichen Silberhalogenidemulsionsschicht, der ein Farbkuppler zugeordnet ist, dadurch gekennzeichnet, daß der Farbkuppler der Formel I entspricht 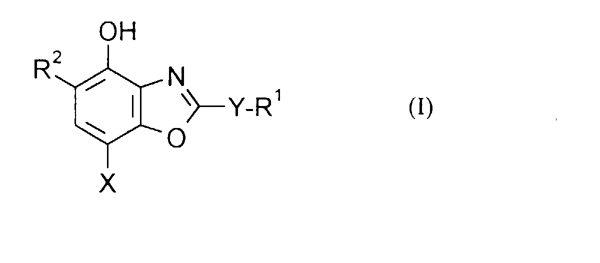 worin bedeuten
R¹, R² ein H-Atom oder einen Substituenten;
X ein H-Atom oder einen sonstigen bei der chromogenen Entwicklung abspaltbaren Rest;
Y eine chemische Bindung, einen Arylenrest, einen Hetarylenrest oder eine der folgenden Gruppen, die über eins ihrer N-Atome an den Benzoxazolring gebunden ist: -NH-CO-, -NH-SO₂-, -NH-CO-NH-, -NH-SO₂-NH-, -NH-CO-O-;
wobei ein durch R¹ oder R² dargestellter Substituent einen Beitrag zur Ballastierung des Cyankupplers und/oder des daraus erzeugten Farbstoffes leistet.

## Claims

1. A photographic colour development process in which a colour photographic recording material having at least one silver halide emulsion layer exposed to form an image is developed in the presence of a colour coupler compound and a dye coupling developer compound, characterised in that the colour coupler compound corresponds to formula I 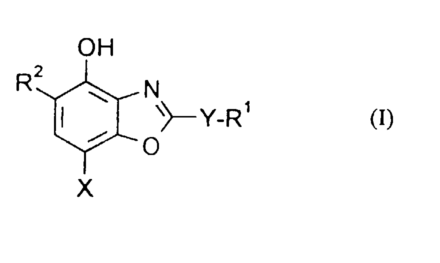 where
R¹, R² represent an H atom or a substituent;
X represents an H atom or another radical which can be split off during chromogenic development: and
Y represents a chemical bond, an arylene radical, a heteroarylene radical or one of the following groups which are bonded to the benzoxazole ring via one of their N atoms: -NH-CO-, NH-SO₂-, -NH-CO-NH-, -NH-SO₂-NH- or -NH-CO-O-;
wherein a substituent represented by R¹ or R² contributes to the ballasting of the cyan coupler and/or of the dye produced therefrom.

2. A colour photographic recording material having at least one light-sensitive silver halide emulsion layer with which a colour coupler is associated, characterised in that the colour coupler corresponds to formula I 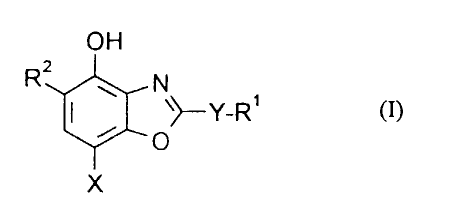 where
R¹, R² represent an H atom or a substituent;
X represents an H atom or another radical which can be split off during chromogenic development; and
Y represents a chemical bond, an arylene radical, a heteroarylene radical or one of the following groups which are bonded to the benzoxazole ring via one of their N atoms: -NH-CO-, NH-SO₂-, -NH-CO-NH-, -NH-SO₂-NH- or -NH-CO-O-;
wherein a substituent represented by R¹ or R² contributes to the ballasting of the cyan coupler and/or of the dye produced therefrom.

## Revendications

1. Procédé photographique de développement en couleurs, dans lequel un matériau d'enregistrement photographique en couleurs avec au oins une couche d'émulsion d'halogénure d'argent impressionnée par une image est développé en présence d'un composé coupleur coloré et d'un composé révélateur chromogène, caractérisé en ce que le composé coupleur coloré correspond à la formule I : 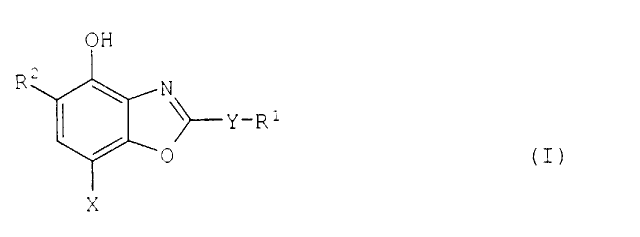 dans laquelle :
R¹, R² représentent un atome de H ou un substituant ;
X représente un atome de H ou un autre résidu dissociable lors du développement chromogène ;
Y représente une liaison chimique, un résidu arylène, un résidu hétéroarylène, ou bien l'un des groupes suivants relié au noyau benzoxazole par l'intermédiaire d'un de leurs atomes de N : -NH-CO-, -NH-SO₂-, -NH-CO-NH-, -NH-SO₂-NH-, -NH-CO-O-,
l'un des substituants représentés par R¹ ou R² contribuant à l'inertage du coupleur cyan et/ou du colorant produit à partir de celui-ci.

2. Matériau d'enregistrement photographique avec au moins une couche d'émulsion d'halogénure d'argent photosensible à laquelle est affecté un coupleur coloré, caractérisé en ce que le coupleur coloré correspond à la formule I : 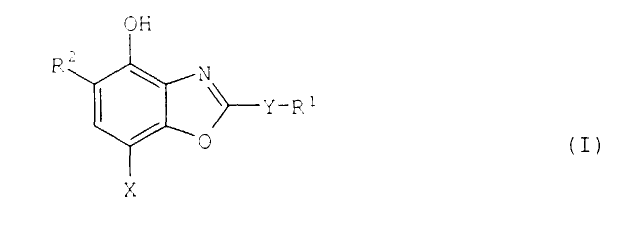 dans laquelle :
R¹, R² représentent un atome de H ou un substituant ;
X représente un atome de H ou un autre résidu dissociable lors du développement chromogène ;
Y représente une liaison chimique, un résidu arylène, un résidu hétéroarylène, ou bien l'un des groupes suivants relié au noyau benzoxazole par l'intermédiaire d'un de leurs atomes de N : -NH-CO-, -NH-SO₂-, -NH-CO-NH-, -NH-SO₂-NH-, -NH-CO-O-,
l'un des substituants représentés par R¹ ou R² contribuant à l'inertage du coupleur cyan et/ou du colorant produit à partir de celui-ci.
